Europäisches Patentamt

European Patent Office

Office européen des brevets

(19)

(11) Numéro de publication: **0 346 244**
**A1**

(12) **DEMANDE DE BREVET EUROPEEN**

(21) Numéro de dépôt: **89401620.3**

(22) Date de dépôt: **12.06.89**

(51) Int. Cl.⁴: **A 61 F 13/06**

(30) Priorité: **10.06.88 FR 8807741**

(43) Date de publication de la demande:
**13.12.89 Bulletin 89/50**

(84) Etats contractants désignés:
**AT BE CH DE ES FR GB GR IT LI LU NL SE**

(71) Demandeur: **Morizet, Philippe**
**33 Avenue Auguste Renoir**
**F-78160 Marly le Roi (FR)**

(72) Inventeur: **Morizet, Philippe**
**33 Avenue Auguste Renoir**
**F-78160 Marly le Roi (FR)**

(54) **Dispositif de contention pour l'articulation de cheville adaptable sur ou dans une chaussure et chaussure ainsi obtenue.**

(57) La présente invention concerne un dispositif de contention adaptable sur ou dans une chaussure et/ou à une semelle, le dispositif de contention comportant une bande (21) de contention en forme de I, ou de V, ou de Y, montée de façon amovible dans ou sur une chaussure et un élément principal indépendant entourant le bas de la jambe, caractérisé en ce que la bande (21) de contention en forme de I, de V ou de Y comporte des moyens de se solidariser à la partie du fond de la chaussure et/ou aux parties latérales de la chaussure, du talon jusqu'au milieu de la chaussure et en ce que les extrémités de la bande (21) de contention en forme de I, ou V ou Y comportent au moins sur la face tournée vers le pied des moyens d'accrochage (200, 210) destinés à s'accrocher sur un élément principal indépendant entourant la jambe au-dessus des malléoles, cet élément principal indépendant comportant des moyens de serrage (100) sur la jambe et d'accrochage des extrémités de la bande dans un "sandwich" dont les couches supérieure et inférieure sont constituées par les parties extrêmes de l'élément principal.

Fig 11

EP 0 346 244 A1

**Description**

## DISPOSITIF DE CONTENTION POUR L'ARTICULATION DE CHEVILLE ADAPTABLE SUR OU DANS UNE CHAUSSURE ET CHAUSSURE AINSI OBTENUE

La présente invention concerne un dispositif de contention pour l'articulation de cheville adaptable sur ou dans une chaussure et la chaussure de contention ainsi obtenue.

Il est connu, notamment par la demande de brevet français 87 07771, déposée au nom du Docteur Philippe MORIZET et intitulée "Dispositif de contention pour l'articulation de cheville et procédé de mise en oeuvre du dispositif", un dispositif de contention utilisant une bande principale destinée à entourer la jambe au niveau de la cheville et à fixer sur cet élément principal les extrémités d'un élément en forme de Y de façon à ce que celle-ci soit prise en tenaille entre une couche de l'élément principal et une seconde couche de l'élément principal. La bande en forme de Y étant destinée à passer sous la voûte plantaire du pied dont on souhaite protéger ou soulager la cheville.

Toutefois, ce dispositif connu, bien que très efficace et nettement supérieur aux dispositifs concurrents, présente comme ces derniers deux inconvénients inévitables :
- un contact direct avec le pied qui peut être source d'échauffement, de transpiration, de frottements minimes, de strictions minimes avec répercution anodine sur la circulation superficielle;
une limitation modérée à minime de l'hyper-extension de la cheville.

Un premier but de l'invention est de proposer, pour la première fois, un dispositif de contention qui permette d'éliminer les inconvénients des dispositifs antérieurs. En particulier l'invention propose un dispositif de contention adaptable aisément sur une chaussure de façon à augmenter l'efficacité de son action qui va s'exercer sur toute toute la longueur du bord externe du pied (du talon au petit orteil).

Ce premier but est atteint par le fait que le dispositif de contention comporte une bande de contention en forme de I, ou de V, ou de Y, montée de façon amovible dans ou sur une chaussure et un élément principal indépendant entourant le bas de la jambe, est caractérisé en ce que la bande de contention en forme de I, de V ou de Y, comporte des moyens de se solidariser à la partie du fond de la chaussure et/ou aux parties latérales de la chaussure, (du talon jusqu'au milieu de la chaussure) en ce que les extrémités de la bande de contention en forme de I, de V ou de Y comportent au moins sur la face tournée vers le pied des moyens d'accrochage destinés à s'accrocher sur l'élément principal indépendant entourant la jambe au-dessus des malléoles, cet élément principal indépendant comportant des moyens de serrage sur la jambe et d'accrochage des extrémités de la bande dans un "sandwich" dont les couches supérieure et inférieure sont constituées par les parties extrêmes de l'élément principal.

Selon un autre but la chaussure peut être conformée de façon à s'adapter immédiatement à l'élément de contention.

Ce but est atteint par le fait que la doublure interne de la chaussure est montée amovible sur les parties latérales, du talon jusqu'au milieu de la chaussure et comporte du velcro sur l'intérieur de la paroi externe et du velcro complémentaire sur l'intérieur de la doublure.

Selon un autre but, on choisira le velcro orienté vers les parties du corps humain de façon à ce qu'il soit le plus confortable.

Ce but est atteint par le fait que les moyens de solidariser la bande de contention sont du velcro doux ou à boucles sur l'intérieur de la paroi externe.

Selon une autre caractéristique, le velcro sur l'intérieur de la doublure est à picots.

Selon une autre caractéristique, le velcro peut s'étendre également sur la partie interne du fond de la chaussure en vis-à-vis du talon.

Un autre but de l'invention est de proposer un dispositif d'adaptation sur une chaussure d'un dispositif de contention caractérisé en ce que le dispositif d'adaptation comprend un élément dont la face supérieure constitue les moyens d'accrochage destinés à coopérer avec la bande de contention en forme de I, de V ou de Y, cet élément étant livré avec des moyens de fixation pour assurer sa fixation avec l'intérieur de la chaussure.

Un autre but est de proposer une semelle intérieure amovible adaptable au dispositif de contention.

Ce but est atteint par le fait que le dispositif de contention pour chaussure est caractérisé en ce qu'il comprend un élément en forme de I, ou de V ou de Y dont la racine de la fourche du V ou du Y ou une extrémité ou partie centrale du I est solidaire de la moitié postérieure d'une semelle intérieure amovible, cette semelle étant rendue solidaire dans sa moitié postérieure, du fond de la chaussure, cet élément étant destiné à coopérer avec un élément principal indépendant entourant la jambe au-dessus des malléoles.

Un autre but de l'invention est de proposer une chaussure directement adaptée pour la contention de la cheville.

Ce but est atteint par le fait que la chaussure comporte sur sa partie latérale située sous la malléole externe une bande solidaire de façon fixe de la chaussure et comportant au moins sur la face tournée vers la jambe des moyens d'accrochage destinés à s'accrocher sur un élément principal indépendant entourant la jambe au-dessus des malléoles et comportant des moyens de serrage sur la jambe et d'accrochage de la bande dans un "sandwich" formé par les parties extrêmes de l'élément principal comportant sur les faces extrêmes opposées des moyens d'accrochage complémentaires.

Selon une caractéristique supplémentaire, la bande est en forme de V et comporte des moyens d'accrochage à chacune des extrémités des branches du V et des moyens d'accrochage de type complémentaire sur les faces opposées de ces branches.

Selon une caractéristique supplémentaire la partie latérale interne de la chaussure comporte une deuxième bande solidaire de façon fixe ou non de la chaussure et comportant au moins sur la face interne tournée vers le pied des moyens d'accrochage sur l'élément principal indépendant.

Selon une autre caractéristique, l'élément en V, ou en Y, ou en I est rendu solidaire en le surmoulant dans la semelle extérieure de la chaussure, ou entre la semelle et le corps de la chaussure.

Selon une autre caractéristique, l'élément en V, en Y ou en I est rendu solidaire par collage, soudure, couture, rivets, fermeture à glissière, laçage.

D'autres caractéristiques et avantages de la présente invention apparaîtront plus clairement à la lecture de la description ci-après faite en référence aux dessins annexés dans lesquels :

- la figure 1 représente une vue de dessus d'une chaussure gauche équipée d'une bande de contention externe en forme de V;

- la figure 2 représente une vue de dessus de la même chaussure gauche équipée d'une bande en forme de V sur le côté extérieur du pied et une bande en forme de I sur le côté intérieur du pied;

- la figure 3 représente une vue en coupe partielle de l'intérieur de la chaussure gauche;

- la figure 4 représente une vue en coupe partielle de l'intérieur de la chaussure gauche lors de la mise en place d'une bande en forme de V amovible;

- la figure 5 représente une vue en coupe partielle de l'intérieur de la chaussure gauche une fois la bande en forme de V en place;

- la figure 6 représente une vue en coupe partielle de l'extérieur de la chaussure gauche après mise en place d'une bande en forme de I;

- la figure 7 représente une vue interne de la partie de la chaussure gauche située à l'extérieur du pied selon une autre variante de l'invention;

- les figures 8A et 8B représentent l'élément principal indépendant, respectivement en vue externe et interne;

- la figure 9 représente la mise en place de l'élément indépendant principal;

- la figure 10 représente la mise en place de la chaussure de contention;

- la figure 11 représente la position à adopter pour la mise en place et en tension des bandes de contention;

- la figure 12 représente une vue du pied gauche du côté extérieur, avant fermeture de l'élément principal sur les bandes de contention;

- la figure 13 représente la chaussure de contention une fois mise en place;

- les figures 14A à 14E représentent différentes variantes de bandes de contention;

- les figures 15A et 15B représentent une variante de réalisation d'un élément d'adaptation pour chaussure.

Les figures 16A, 16B, 16C, 16D représentent une semelle de chaussure adaptée avec le dispositif de contention.

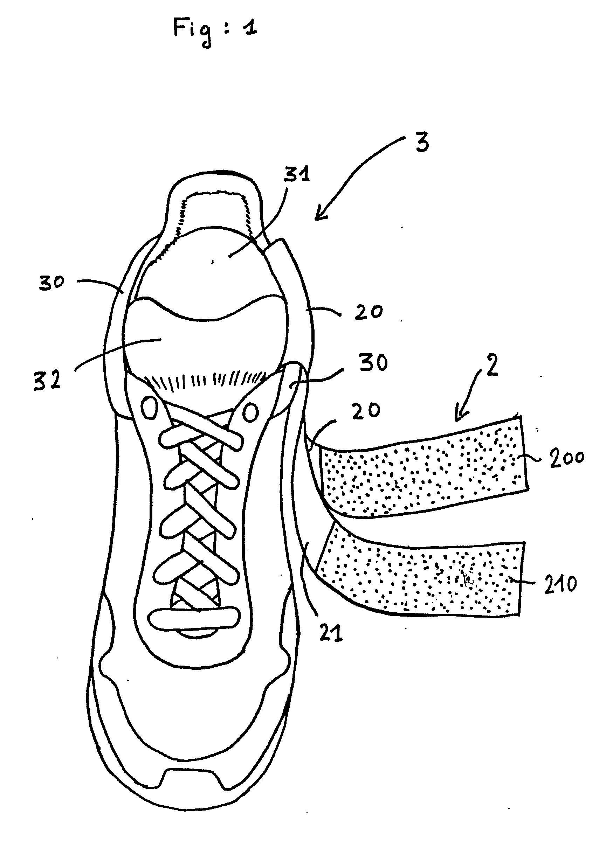

La figure 1 représente une vue de dessus d'une chaussure gauche équipée d'une languette (32) de protection du coup de pied et d'une bande de contention en forme de V dont la première branche (20) comporte à son extrémité du velcro à crochets (200) sur la face tournée vers la jambe et dont la deuxième branche (21) comporte à son extrémité du velcro à crochets (210) sur la face tournée vers la jambe. Cette bande de contention (20) peut faire partie intégrante de la chaussure ou être reliée de manière fixe ou amovible à chaussure, de façon à être solidaire de la partie latérale de la chaussure entre le talon (30) et le milieu de la chaussure. Sur la face opposée au velcro à crochets (200, 210) se trouve du velcro à boucles (201, 211).

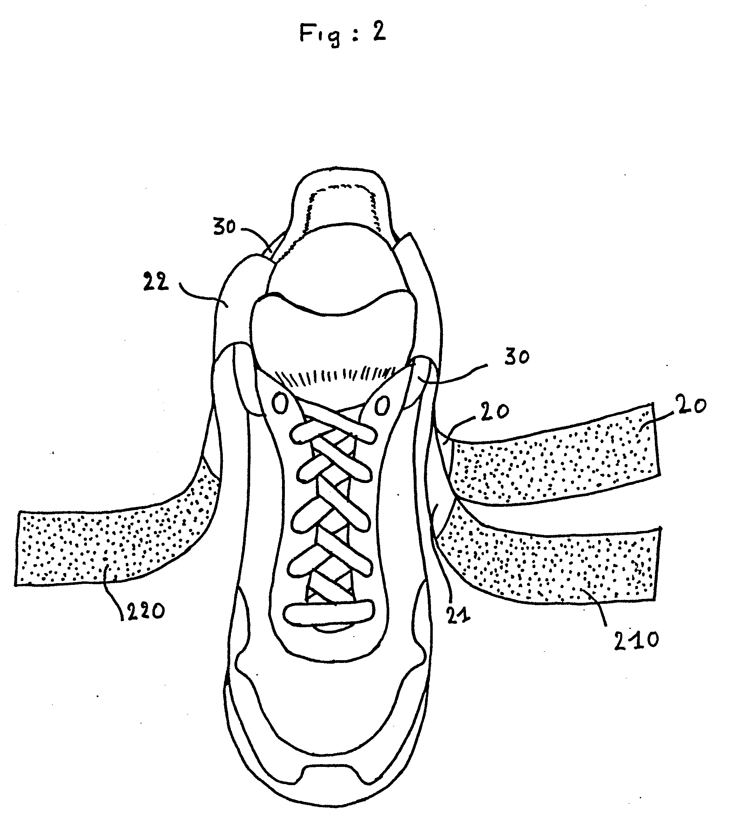

La figure 2 représente la même chaussure avec, en plus de la bande de contention en V une bande en forme de I, (22) dont l'extrémité éloignée de la chaussure comporte du velcro à crochets (220) sur la face tournée vers la jambe. Cette bande (22) en forme de I, comme on le verra par la suite, peut faire partie intégrante de la chaussure ou être reliée de manière fixe ou amovible à la chaussure, ou à la bande en forme de V de façon à former une bande en forme de Y solidaire de la bande de contention en forme de V de façon à former une bande en forme de Y. Sur la face opposée au velcro à crochets (220) se trouve du velcro à boucles (221).

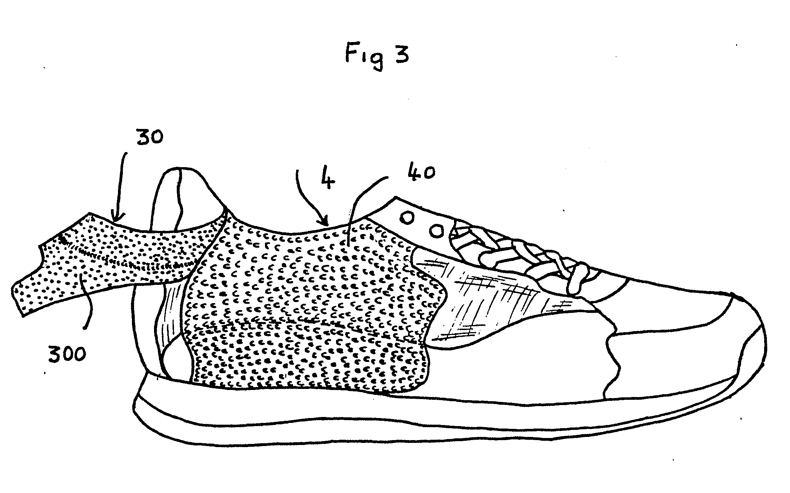

La figure 3 représente une vue en écorché de la paroi interne de la partie latérale externe (4) d'une chaussure gauche lorsque la doublure interne ou bourrelet (30) de protection et la semelle intérieure sont enlevés. Cette paroi interne (4) comporte une pièce en tissu velcro à boucles (40) ou velcro doux, rendu solidaire par collage, soudure, couture, ou rivetage, à la fois de la paroi latérale (4) située au dessous de la malléole externe et de la portion du fond de la chaussure située sous le talon du pied. La paroi interne de la doublure (30) comporte du velcro à crochets (300).

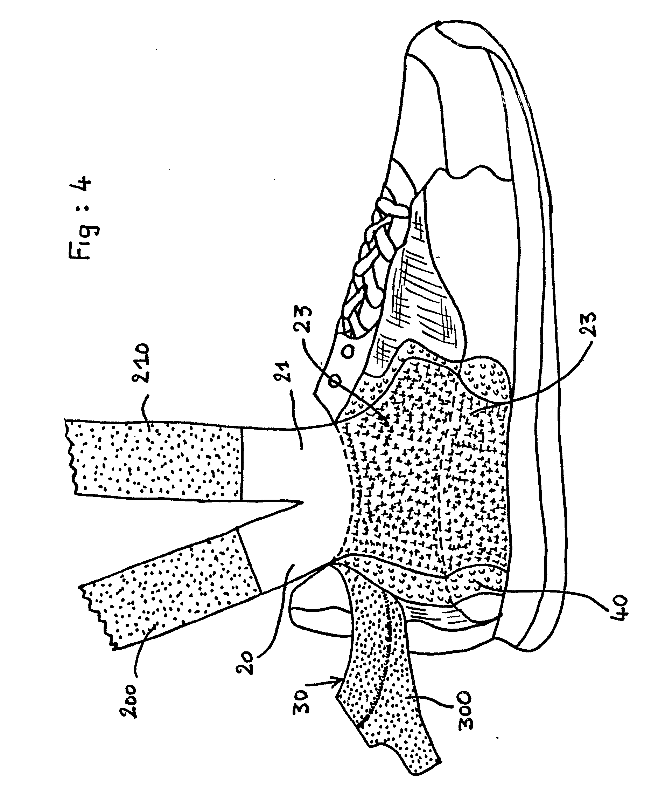

La figure 4 représente une vue en écorché de la même chaussure gauche avec accrochée sur le velcro à boucle (40) la racine (23) d'une bande de contention en forme de V dont les branches (21) et (20) sont constituées de la façon décrite antérieurement. La racine (23) comporte sur la face tournée vers l'observateur, du velcro doux sur lequel vient s'accrocher le velcro à crochets (300) du bourrelet, tandis que la face de la racine (23) tournée vers le velcro à boucles (40) est constituée par du velcro à crochets. De cette façon la racine de la bande en forme de V est accrochée sur l'intérieur de la garniture de la chaussure, que ce soit sur les parois latérales ou sur le fond de la chaussure et cette racine (23) est prise en tenaille ou "sandwich" par le bourrelet ou doublure intérieur (30) de protection.

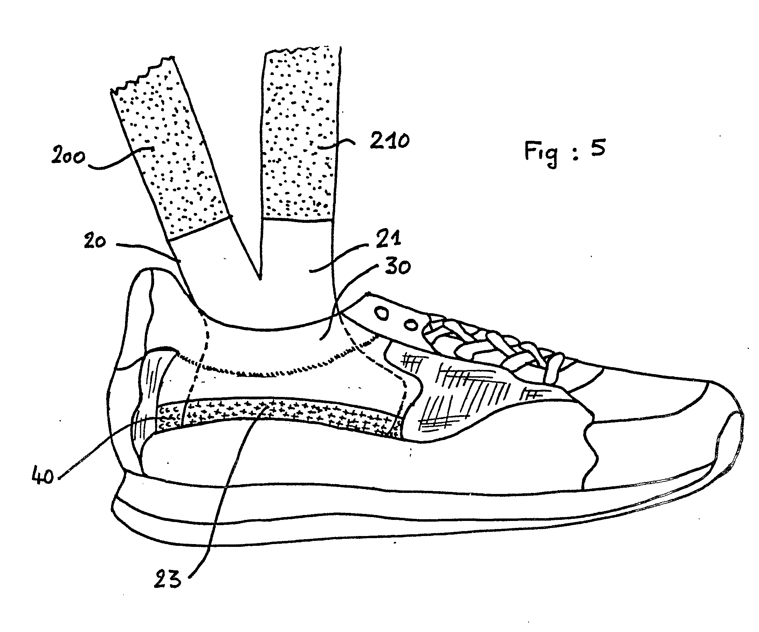

La figure 5 représente une vue en écorché de la chaussure équipée selon la figure 4 une fois que la garniture intérieure du bourrelet (30) est rabattue sur la racine (23) de la bande en V.

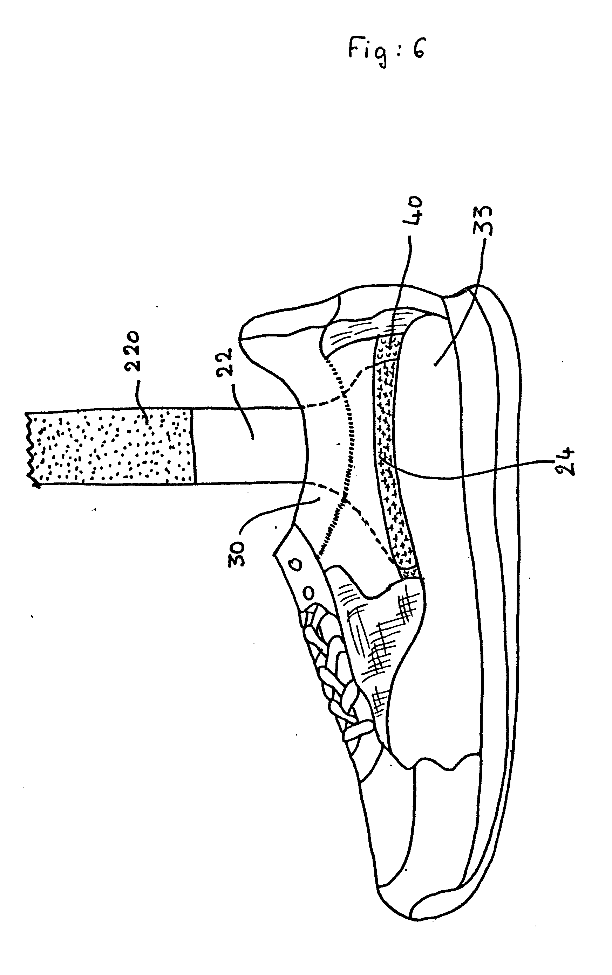

La figure 6 représente un mode de réalisation simplifié d'une chaussure de contention dans lequel on se contente simplement de rapporter une bande en forme de I (22) comportant à son extrémité éloignée de la chaussure et tournée vers la jambe du velcro à crochets (220) et sur la face opposée de cette extrémité du velcro à boucles (221). La racine (24) de cette bande en forme de I comporte sur la face tournée vers l'intérieur du velcro à boucles et sur la face tournée vers la paroi interne de la chaussure du velcro à crochets. Ce velcro à crochets est destiné à se fixer sur la paroi interne (4) constituée par du velcro à boucles (40). Sur la partie à boucles (24) on rabat la languette (30) dont les crochets situés à l'intérieur viennent se fixer.

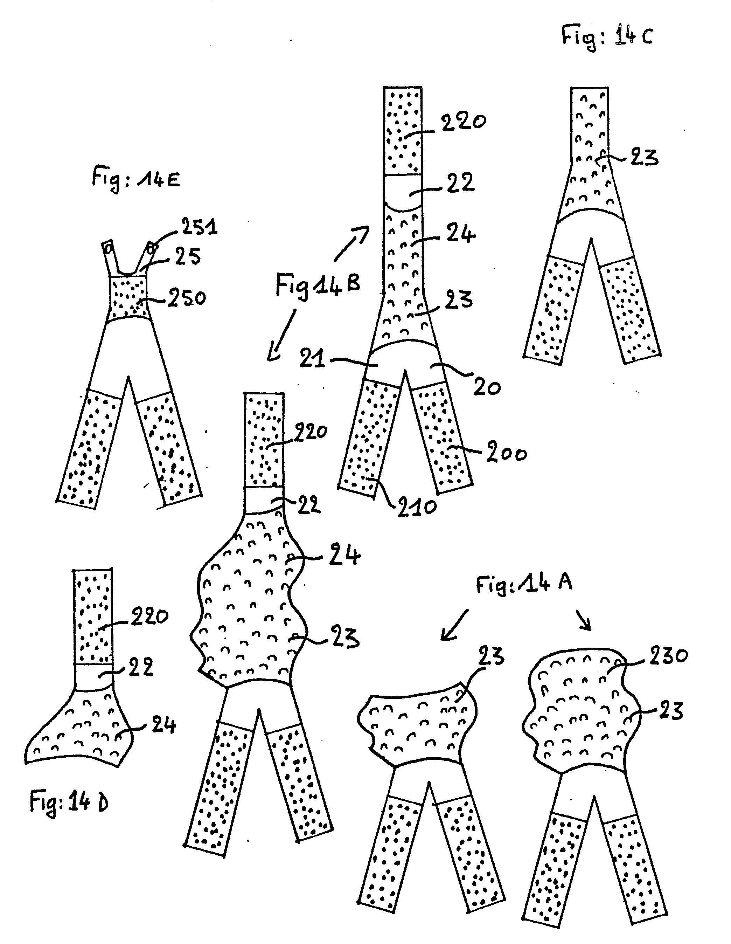

Cette bande en forme de I peut être utilisée isolément, côté interne ou externe de la chaussure, soit en combinaison avec une bande en forme de V, disposée sur le côté interne ou externe de la chaussure. On peut également constituer une seule pièce avec une bande en forme de V et une bande en forme de I de façon à former une bande de contention en forme de Y, comme on peut le voir sur la figure 14.

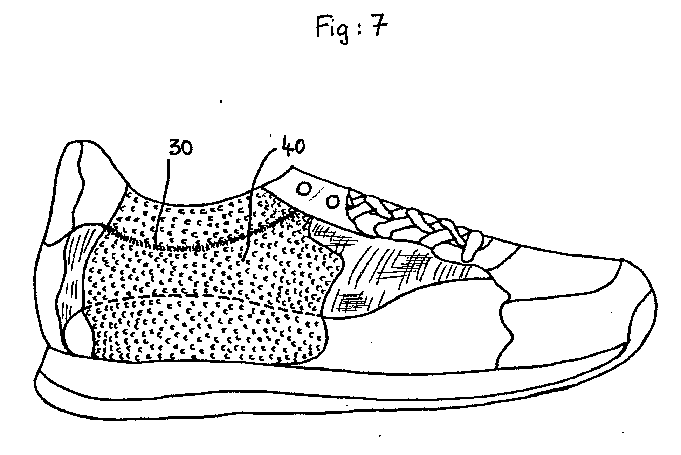

La figure 7 représente une variante de réalisation d'une chaussure équipée d'un dispositif d'adaptation pour bande de contention, cette chaussure dont on a enlevé la semelle intérieure est équipée d'un élément (40) constitué par du velcro à boucles rendu solidaire par collage, soudure ou couture de la paroi latérale de la chaussure et éventuellement de la partie du fond de chaussure situé sous le talon du pied. Dans cette variante, l'élément (40) est directement rapporté sur le bourrelet intérieur (30) que l'on peut voir en pointillés. Dans ce cas l'élément de contention viendra directement s'agripper par ses crochets sur l'élément (40) mais ne sera pas pris en tenaille par le bourrelet intérieur (30). La semelle intérieure, une fois remise en place contribue au maintien de l'accrochage de la bande de contention sur le fond de la chaussure.

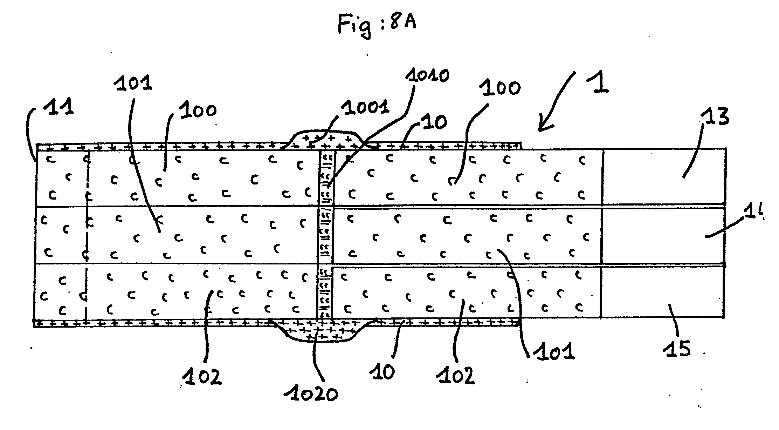

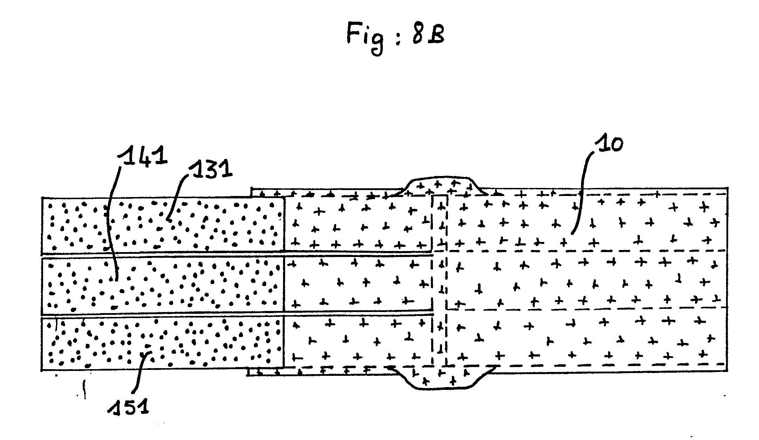

Les figures 8A et 8B représentent l'élément principal indépendant (1) constitué par un matériau à la fois souple, plus ou moins rigide et inextensible, tel que, par exemple, de la toile, se prolongeant par des languettes (13, 14, 15) et dont la face interne est recouverte par du tissu éponge ou autre matériau de protection (10) sur une distance correspondant au périmètre moyen d'une cheville et sur le restant de la longueur de l'élément principal par des bandes velcro à crochets (131, 141, 151). La face externe de l'élément principal comporte du tissu velcro doux monté éventuellement en bandes (100, 101, 102) et s'étendant sur une longueur supérieure à la longueur de la partie en éponge (10). Au niveau du talon ou du tendon d'achille, l'élément principal comporte des bourrelets (1020, 1001) protégeant ce dernier et une bande élastique (1010) permettant une pose de l'élément principal plus facile, qui peut s'adapter ainsi à n'importe quelle forme de cheville.

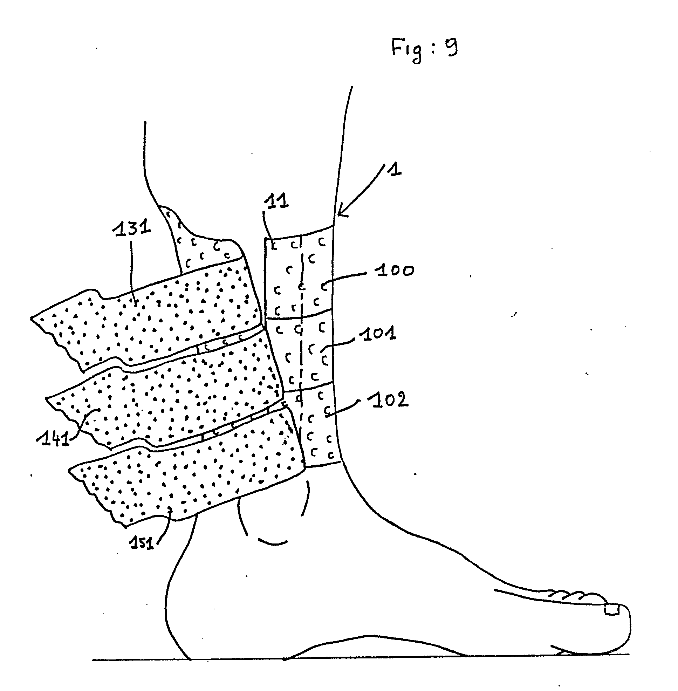

La mise en place du dispositif décrit ci-dessus va être explicité à l'aide des figures 9 à 13.

Sur la figure 9 on voit la mise en place de l'élément principal indépendant (1) au-dessus des malléoles de la cheville avec l'extrémité (11) de l'élément principal situé à la verticale de la malléole interne. Les surfaces à picots (131, 141, 151) des bandes (13, 14, 15) viennent s'agripper sur le velcro à boucles (100, 101, 102) jusqu'au niveau du tibia.

Une fois l'élément principal fixé sur la cheville, la chaussure (3) est chaussée et lacée. Eventuellement, avant d'enfiler la chaussure, on a passé une chaussette, directement au contact de la peau, auquel cas l'élément principal (1) se trouve sur la chaussette. Si on utilise une chaussette spéciale ayant, au niveau de la jonction entre la zone (221) et la zone (22) une fente qui permet de passer la languette (22) à l'extérieur de la chaussette par cette fente et de l'autre côté la bande en forme de V à l'extérieur de la chaussette, la chaussette est rabattue sur le coup de pied pour la mise en place de l'élément principal (1) et la fixation de la bande en forme de I sur l'élément principal (1) et la bande en V. Il peut également exister un autre type de chaussette (avec rabat sur le dispositif par exemple).

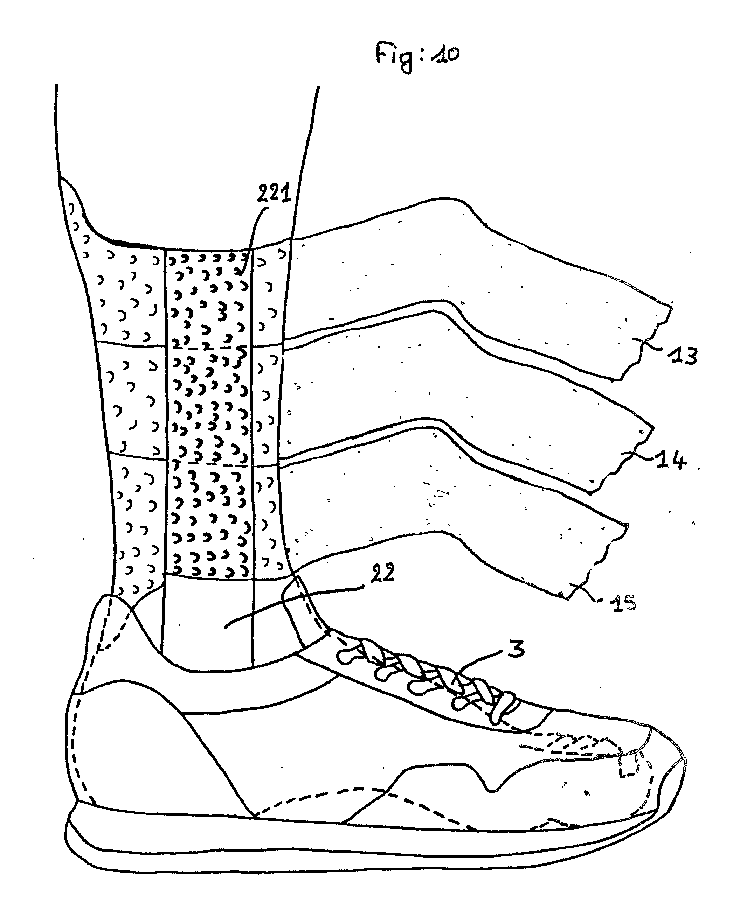

La figure 10 montre la mise en place de l'élément en forme de I sur la jambe gauche ou de la bande de contention en forme de Y lorsque la chaussure est équipée de façon amovible ou non d'un tel élément. La surface (220) à crochets de cet élément (22) s'accroche sur le velcro à boucles des parties (100, 101, 102) au-dessus de la malléole interne. La face opposée à la surface (220) est constituée par du velcro à boucles (221).

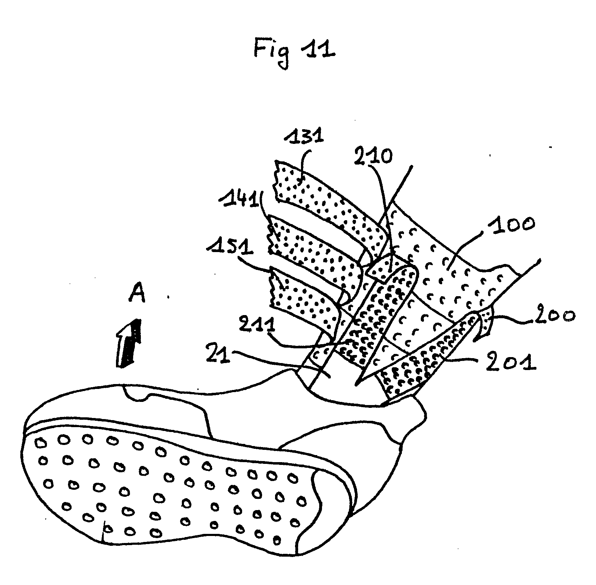

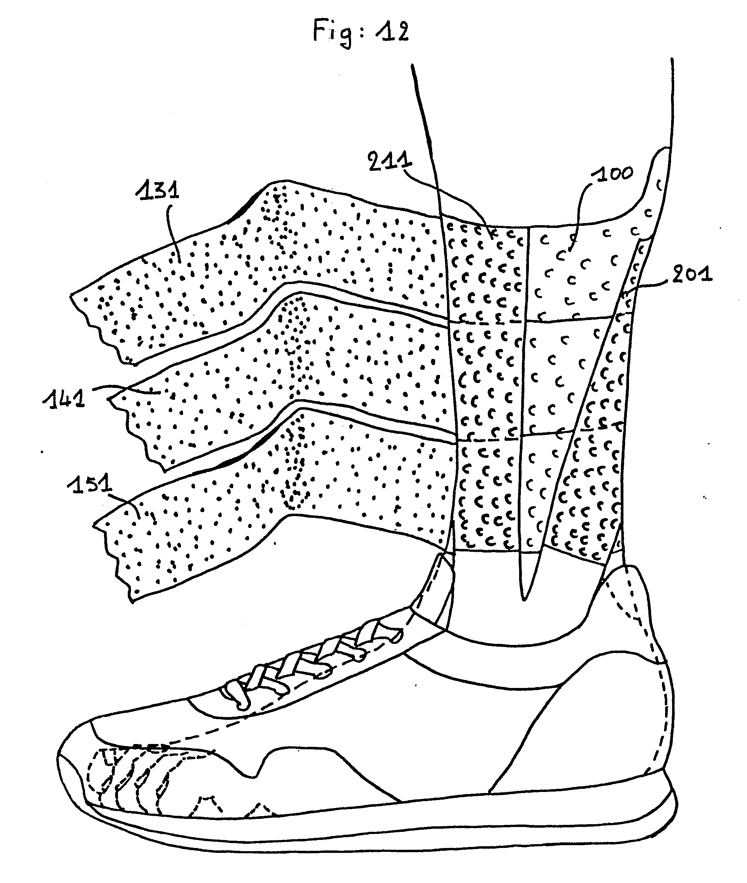

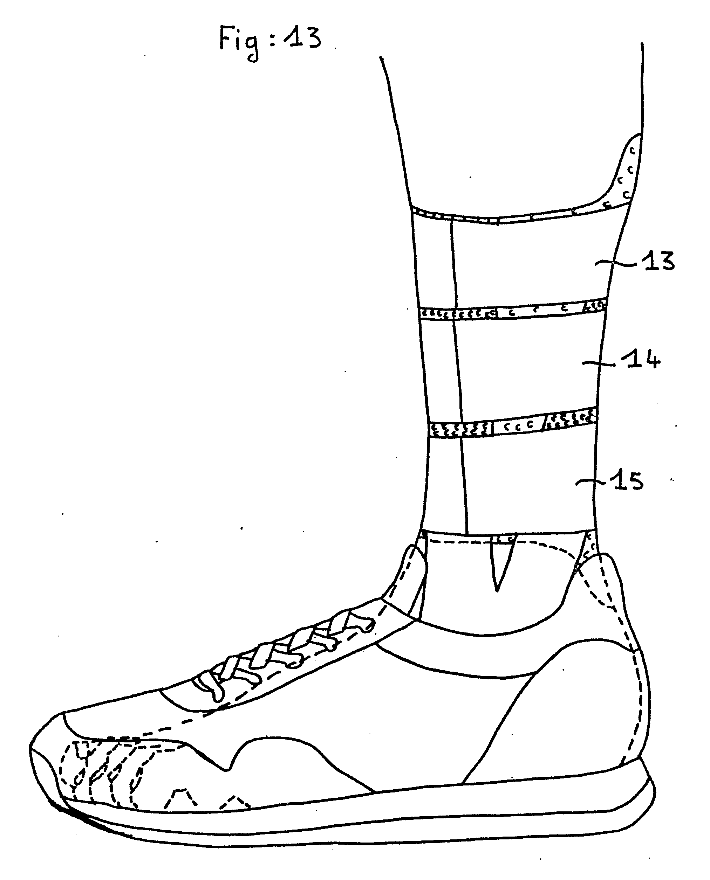

La figure 11 représente la mise en place de l'élément de bande de contention en forme de V après avoir mis le pied en position valgus, en relevant la pointe du pied en haut et en dehors dans un mouvement d'anti-torsion du ligament latéral externe, comme représenté par la flèche (A) sur la figure 11. Le pied étant maintenu dans cette position, les bandes (21) et (20) sont accrochées sur le velcro à boucles (100 à 102) grâce au velcro à crochets (210, 200), figure 1, sur les faces internes de ces bandes (21, 20). Les faces opposées à ces faces internes comportent des zones (211, 201) comportant du velcro à boucles. Une fois les bandes (21, 20) en forme de V appliquées comme représenté à la figure 12, on rabat les languettes (13, 14, 15) pour appliquer les faces à velcro à crochets (131, 141, 151) sur le velcro à boucles (211, 201) des bandes (21, 20) et sur le velcro à boucles (100 à 102) de l'élément principal. Comme représenté à la figure 13, on enroule les bandes (13, 14, 15) autour de la cheville de manière à ce qu'elles viennent recouvrir le velcro à boucles (221) de la bande (22) formant l'extrémité d'une bande de contention en forme de Y ou d'une bande de contention en forme de I indépendante de la bande en forme de V. Les bandes (21, 22) ayant été passées au travers de la chaussette, une fois le dispositif de contention mis en place, comme représenté à la figure 13, on peut remonter la chaussette le long du mollet et celle-ci recouvre le dispositif. Seule du côté externe, la bande (21) et (20) passe à travers la fente vers l'intérieur de la chaussette et se trouve visible et à l'intérieur du pied, la bande (22) se trouve visible au niveau de l'arrondi de la chaussure.

La figure 14A représente une bande de contention en forme de V comportant une racine (23) destinée à être solidarisée de la paroi latérale et dans cette variante une racine (23) prolongée par une zone (230) destinée à être placée sous le talon du pied, sous la semelle de la chaussure.

La figure 14B représente deux variantes de bande de contention en forme de Y, l'une avec des zones (23, 24,) plus larges de manière à améliorer la solidarisation avec la partie interne de la chaussure. Les deux variantes comportent une bande (22) qui se prolonge pour venir se fixer, en protégeant le ligament latéral interne, sur le côté interne de l'élément principal indépendant.

La figure 14C représente une variante de bande de contention en forme de Y dont seule la racine (23) constitue la jambe centrale du Y, cette jambe centrale (23) venant se placer sous le talon dans la chaussure.

La figure 14D représente une forme de réalisation de bande de contention indépendante en forme de I avec une racine (24) élargie au niveau du talon du pied pour améliorer sa fixation sur la chaussure. Cette réalisation peut être utilisée et rajoutée à une chaussure déjà équipée d'une bande de contention selon la variante de la figure 14A.

Enfin la figure 14E représente une dernière variante de réalisation de bande de contention en forme de V dont la racine comporte au moins une languette (25) avec une ouverture (251) destinée à venir se fixer autour d'un crampon (ou sur sa vis) de chaussure de sport. Cette racine (25) peut également comporter une zone (250) à velcro à crochets destinée à venir s'accrocher sur une partie de la paroi extérieure de la chaussure équipée de velcro à boucles.

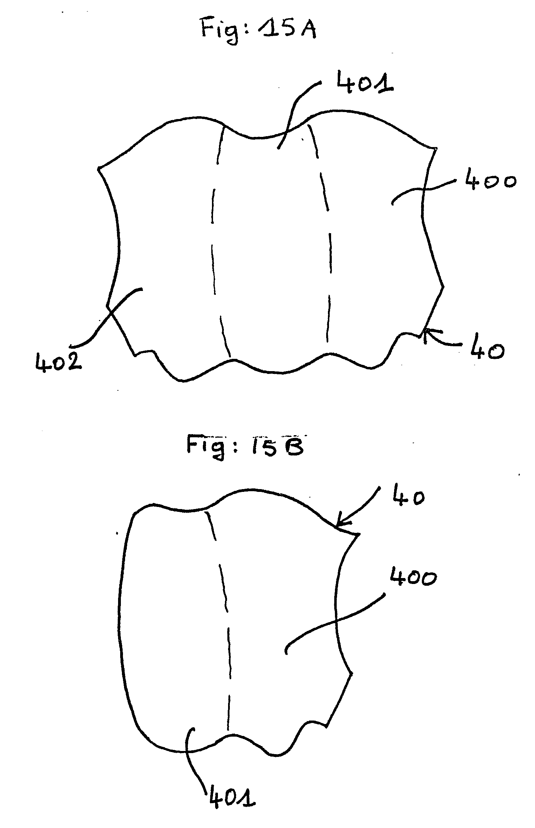

La figure 15A représente un premier type de dispositif d'adaptation pour chaussure destiné à être utilisé avec un dispositif de contention. Ce dispositif d'adaptation est constitué par une pièce (40) dont les parties latérales (400, 402) et supérieures sont constituées par du velcro doux et la partie centrale (401) également en velcro doux est destinée à être placée au niveau du talon. Cette pièce (40) est fixée à la chaussure par soudure ou collage, ou tout autre moyen approprié et permet d'accrocher sur les parois latérales et le fond de la chaussure la bande de contention en forme de V, de Y ou de I destinée à coopérer avec l'élément principal indépendant.

La figure 15B représente un deuxième mode de réalisation destiné simplement à être utilisé principalement avec une bande de contention en forme de V ou de I. Dans cette variante, l'élément (40) comporte seulement un côté latéral (400) et une partie centrale (401) qui vient se placer sur le fond de la chaussure.

La figure 16A représente une semelle (5) équipée de façon fixe ou amovible d'une bande de contention en forme de Y.

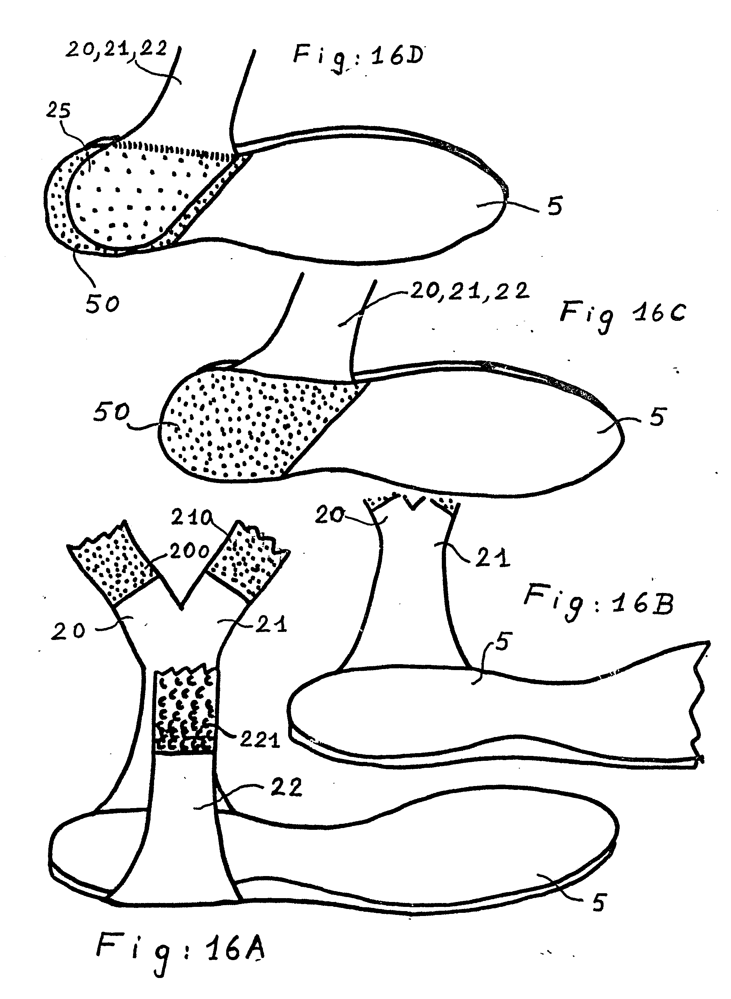

La figure 16B représente la même semelle (5) équipée seulement d'une seule bande de contention en forme de V.

La figure 16C représente une vue du dessous de la semelle (5) selon un premier mode de réalisation dans lequel la bande de contention (20, 21 ou 22) est solidarisée à la semelle de façon fixe. La moitié postérieure de cette semelle est équipée d'un velcro à crochets (50).

La figure 16D représente une vue du dessous d'une semelle (5) selon un deuxième mode de réalisation dans lequel la bande de contention (20, 21 ou 22) est solidarisée à la semelle (5) de façon amovible par l'intermédiaire d'un velcro à crochets (50) fixé sur la moitié postérieure de la face inférieure de la semelle et qui est complémentaire à celui fixé sur la racine de la bande de contention (20, 21, 22). La face opposée (25) de la racine comporte du velcro à crochets (50) complémentaire au velcro doux placé dans le fond de la chaussure et en regard.

On comprend que l'on a ainsi décrit un dispositif de contention facilement adaptable sur ou dans une chaussure, les moyens d'adapter le dispositif de contention à la chaussure et la façon de réaliser une chaussure de contention. Un tel dispositif présente l'avantage d'agir au niveau de la totalité du pied et donc d'être beaucoup plus efficace. Par ailleurs, comme, pour la première fois dans un tel système, il n'y a aucun contact direct entre le pied et les différentes bandes de contention, il n'y a plus les inconvénients de frottement, striction, échauffement, sudation existants avec les dispositifs antérieurs connus. De plus le système présente l'avantage d'être très confortable et de ne pas limiter l'hyper-extension de la cheville, ce qui est très important, notamment pour la pratique des sports. Enfin le dispositif est réajustable très vite et permet rapidement de retendre les bandes en cours de match. D'autre part, si les deux chaussures sont équipées à l'intérieur de velcro doux ou de tout autre dispositif permettant l'attache rapide des ou de la bande de contention, il suffit alors de se procurer une seule bande de contention et un seul élément principal indépendant pour traiter alternativement la cheville gauche ou droite. Enfin la semelle constitue en autre dispositif de contention plus rapidement et plus facilement adaptable à n'importe quelle chaussure.

Toute modification à la portée de l'homme de métier fait également partie de l'esprit de l'invention.

Ainsi, la ou les bandes de contention en I et V peuvent être réalisées en deux portions, une première portion faisant partie intégrante de la chaussure, fixe et une deuxième portion pouvant se solidariser à la première par un système d'attache amovible solide (fermeture à glissière, lacets, rivets, velcro).

**Revendications**

1) Dispositif de contention adaptable sur une chaussure, le dispositif de contention comportant une bande de contention en forme de I, ou de V, ou de Y, montée de façon amovible dans ou sur une chaussure et un élément principal indépendant entourant le bas de la jambe, caractérisé en ce que la bande de contention en forme de I, de V ou de Y comporte des moyens de se solidariser à la partie du fond de la chaussure et/ou aux parties latérales de la chaussure, du talon jusqu'au milieu de la chaussure et en ce que les extrémités de la bande de contention en forme de I, ou V ou Y comportent au moins sur la face tournée vers le pied des moyens d'accrochage destinés à s'accrocher sur un élément principal indépendant entourant la jambe au-dessus des malléoles, cet élément principal indépendant comportant des moyens de serrage sur la jambe et d'accrochage des extrémités de la bande dans un "sandwich" dont les couches supérieure et inférieure sont constituées par les parties extrêmes de l'élément principal.

2) Dispositif de contention selon la revendication 1, caractérisé en ce qu'une doublure interne de la chaussure est montée amovible sur les parties latérales du talon jusqu'aù milieu de la chaussure et comporte du velcro sur l'intérieur de la paroi externe et du velcro complémentaire sur l'intérieur de la doublure.

3) Dispositif selon la revendication 2, caractérisé en ce que le velcro peut s'étendre également sur la partie interne du fond de la chaussure en vis-à-vis du talon.

4) Dispositif d'adaptation sur une chaussure d'un dispositif de contention caractérisé en ce que le dispositif d'adaptation comprend un élément dont la face supérieure constitue les moyens d'accrochage destinés à coopérer avec la bande de contention en forme de I, de V ou de Y, cet élément étant livré avec des moyens de fixation pour assurer sa fixation avec l'intérieur de la chaussure.

5) Dispositif de contention pour chaussure caractérisé en ce qu'il comprend une bande de contention en forme de I, ou de V ou de Y dont la racine de la fourche du V ou du Y ou une extrémité ou partie centrale du I est solidaire, de façon amovible ou non, de la moitié postérieure d'une semelle intérieure amovible, cette semelle étant rendue solidaire, de façon amovible ou non, dans sa moitié postérieure du fond de la chaussure, cette bande étant destinée à coopérer avec un élément principal indépendant entourant la jambe au-dessus des malléoles.

6) Chaussure adaptée pour la contention de la cheville caractérisé en ce que la chaussure comporte sur la partie latérale de la chaussure située sous la malléole une bande solidaire de façon fixe de la chaussure et comportant au moins sur la face tournée vers la jambe des moyens d'accrochage destinés à s'accrocher sur un élément principal indépendant entourant la jambe au-dessus des malléoles et comportant des moyens de serrage sur la jambe et d'accrochage de la bande dans un "sandwich" formé par les parties extrêmes de l'élément principal comportant sur les faces extrêmes opposées des moyens d'accrochage complémentaires.

7) Chaussure selon la revendication 6, caractérisée en ce que la bande est en forme de V et comporte des moyens d'accrochage à chacune des extrémités des branches du V et des moyens d'accrochage de type complémentaire sur les faces opposées de ces branches.

8) Chaussure selon la revendication 6 ou 7, caractérisée en ce que la partie latérale interne de la chaussure comporte une deuxième bande solidaire de façon fixe ou non de la chaussure et comportant au moins sur la face interne tournée vers le pied des moyens d'accrochage sur l'élément principal indépendant.

9) Chaussure selon la revendication 6, caractérisée en ce que l'élément en V, ou en Y, ou en I est rendu solidaire en le surmoulant dans la semelle extérieure de la chaussure ou entre la semelle et le corps de la chaussure.

10) Chaussure selon la revendication 6, caractérisée en ce que l'élément en V, en Y ou en I est rendu solidaire par collage, soudure, couture, fermeture à glissière, rivet, laçage.

11) Chaussure de contention selon une des revendications 6 à 10, caractérisée en ce que la ou les bandes solidaires de la chaussure comportent une première portion faisant partie intégrante de la chaussure et une deuxième portion comportant les moyens d'accrochage à l'élément principal et des moyens de solidarisation à la première portion.

12) Chaussure ou dispositif selon une des revendications précédentes, caractérisé en ce que l'élément principal indépendant comporte au niveau du talon d'achille une bande élastique (1010).

Fig : 1

Fig : 2

Fig 3

Fig : 4

EP 0 346 244 A1

200    210    Fig : 5

20    21
30

40

23

EP 0 346 244 A1

Fig: 6

Fig : 7

Fig:8A

Fig : 8B

Fig : 9

Fig: 10

Fig 11

Fig: 12

Fig : 13

13

14

15

Fig: 14 C

Fig: 14 E

Fig 14 B

Fig: 14 A

Fig: 14 D

Fig: 15A

401

400

402

40

Fig: 15B

40

400

401

20,21,22

Fig: 16D

25

5

50

20,21,22

Fig 16C

50

5

210

200

20

21

Fig: 16B

20

21

5

221

22

5

Fig: 16A

5

Office européen
des brevets

# RAPPORT DE RECHERCHE EUROPEENNE

Numero de la demande

EP  89 40 1620

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | GB-A-2 162 755  (IVANY) <br> * Résumé; figures * | 1 | A 61 F  13/06 |
| A |  | 6,7,9, 10 | |
| | --- | | |
| X | DE-U-8 701 648  (SCHIEVINK) <br> * Figures; page 2, ligne 1 - page 3, ligne 17 * | 5 | |
| | --- | | |
| A | FR-A-2 345 098  (RABISCHONG) | | |
| | --- | | |
| A | US-A-1 351 248  (HILL) | | |
| | --- | | |
| E | US-A-4 753 229  (SUTHERLAND) <br> * Figures; colonne 2, ligne 42 - colonne 3, ligne 64 * | 1,5 | |
| | ----- | | |

DOMAINES TECHNIQUES RECHERCHES (Int. Cl.4)

A 61 F
A 43 B
A 43 C

Le présent rapport a été établi pour toutes les revendications

| Lieu de la recherche | Date d'achèvement de la recherche | Examinateur |
|---|---|---|
| LA HAYE | 14-09-1989 | STEENBAKKER J. |